# EUROPEAN PATENT SPECIFICATION

(11) **EP 1 787 584 B1**
(45) Date of publication and mention of the grant of the patent: **18.07.2012**
(21) Application number: 06255890.3
(22) Date of filing: 17.11.2006
(51) Int. Cl.: A61B 5/151

(54) **Cap with revolving body for dermal tissue lancing device**
Kappe mit Drehkörper für Hautgewebe-Lanzettenvorrichtung
Capuchon doté d'un corps rotatif pour autopiqueur de tissus dermiques

(30) Priority: 17.11.2005 US 282494
(43) Date of publication of application: 23.05.2007
(73) Proprietor: LifeScan, Inc., Milpitas, CA 95035 (US)
(72) Inventor: Olson, Lorin P., Scotts Valley, CA 95066 (US)
(74) Representative: Tunstall, Christopher Stephen

(56) References cited:
- EP-A1- 1 527 737
- WO-A-2004/045375
- US-A1- 5 951 493
- US-A1- 2006 089 566
- US-B1- 6 589 260

## Description

### BACKGROUND OF THE INVENTION

1. Field of the Invention

The present invention relates, in general, to medical devices and, in particular, to caps for dermal tissue lancing devices.

2. Description of the Related Art

Conventional dermal tissue lancing devices generally have a rigid housing and a lancet that can be armed and launched so as to briefly protrude from one end of the lancing device. For example, conventional lancing devices can include a lancet that is mounted within a rigid housing such that the lancet is movable relative to the rigid housing along a longitudinal axis thereof. Typically, the lancet is spring loaded and launched, upon release of the spring, to penetrate (i.e., "lance") a target site (e.g., a dermal tissue target site on a user's fingertip). A biological fluid sample (e.g., a whole blood sample) can then be expressed from the penetrated target site for collection and analysis. Conventional lancing devices are described in U.S. Patent No. 5,730,753 to Morita, U.S. Patent No. 6,045,567 to Taylor et al. and U.S. Patent No. 6,071,250 to Douglas et al. WO-A-2004-45375 figure 16 discloses a cap for use with a dermal lancing device where the cap has several bendable portions.

Dermal tissue lancing devices often include a cap that engages the target site. Such a cap typically has an aperture (i.e., opening), through which the lancet protrudes, and a distal end of the cap will be placed in contact with the target site during use.

When a cap is contacted with a target site, pressure is usually applied to the target site prior to launch of the lancet. This pressure urges the cap against the target site and creates a target site bulge within the opening of the cap. The lancet is then launched to penetrate the target site bulge. A fluid sample, typically blood, is then expressed from the lanced target site for testing. For example, a blood sample expressed from a lanced dermal tissue target site may be tested for the analyte glucose.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings in which like numerals indicate like elements, objects and forces, of which:

FIG. 1 is a simplified perspective view of a cap for use with a dermal tissue lancing device according to an exemplary embodiment of the present invention;

FIG. 2 is a simplified exploded perspective view of the cap of FIG. A;

FIG. 3 is a simplified perspective, partially-cut-away view of the cap of FIG. 1;

FIG. 4 is a simplified, perspective, partially-cut-away view of the cap of FIG. 1A urged against a dermal tissue target site;

FIG. 5 is a simplified perspective view of a cap for a dermal tissue lancing device cap according to another exemplary embodiment of the present invention;

FIG. 6 is a simplified top view of the cap of FIG. 5;

FIG. 7 is a simplified, perspective, partially-cut-away view of the cap of FIG. 5 with a dashed line depicting a circular axis of the cap's cap body;

FIG. 8 is a simplified perspective, partially-cut-away view of the cap of FIG. 5 urged against a dermal tissue target site;

FIG. 9 is a flow diagram illustrating a sequence of steps in a process according to an exemplary embodiment of the present invention; and

FIGs. 10A, 10B and 10C are perspective, partially-cut-away views depicting various stages of the process of FIG. 9.

### DETAILED DESCRIPTION OF PREFERRED EMBODIMENTS

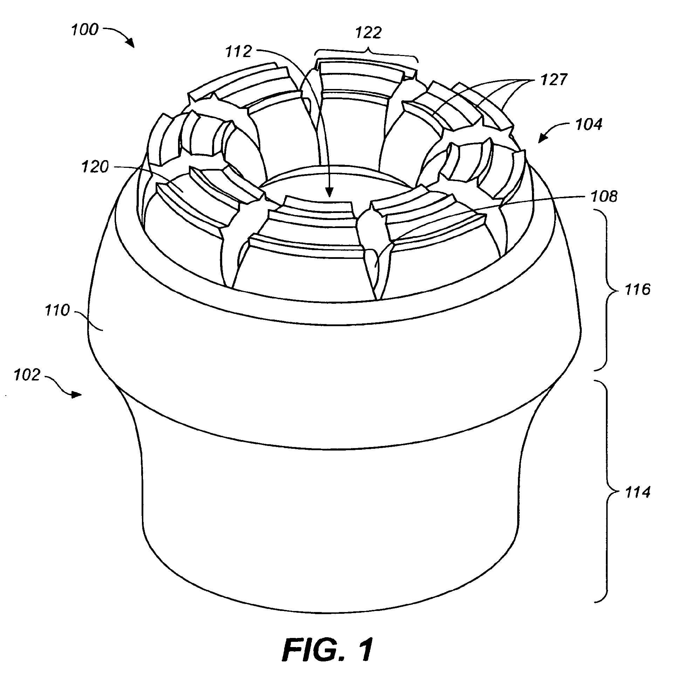

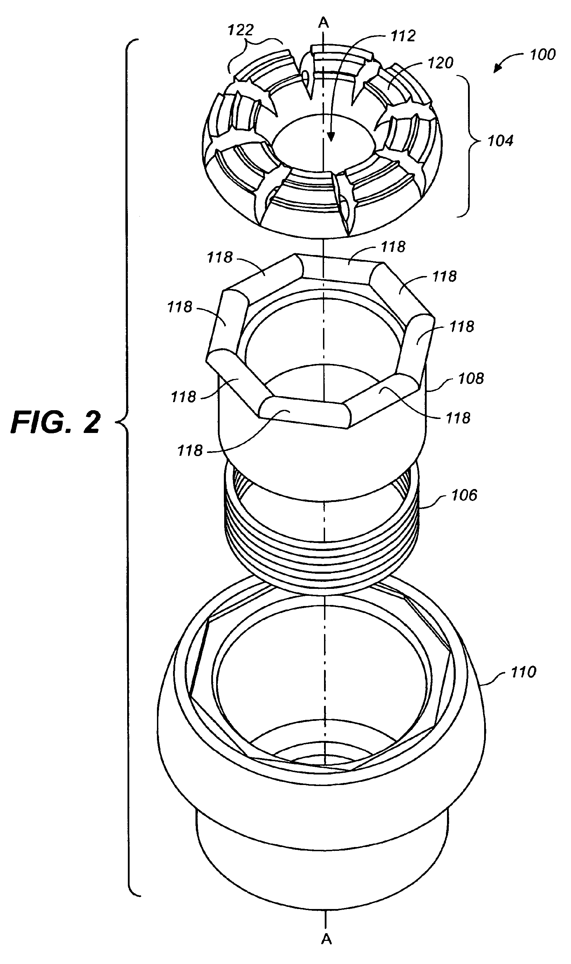

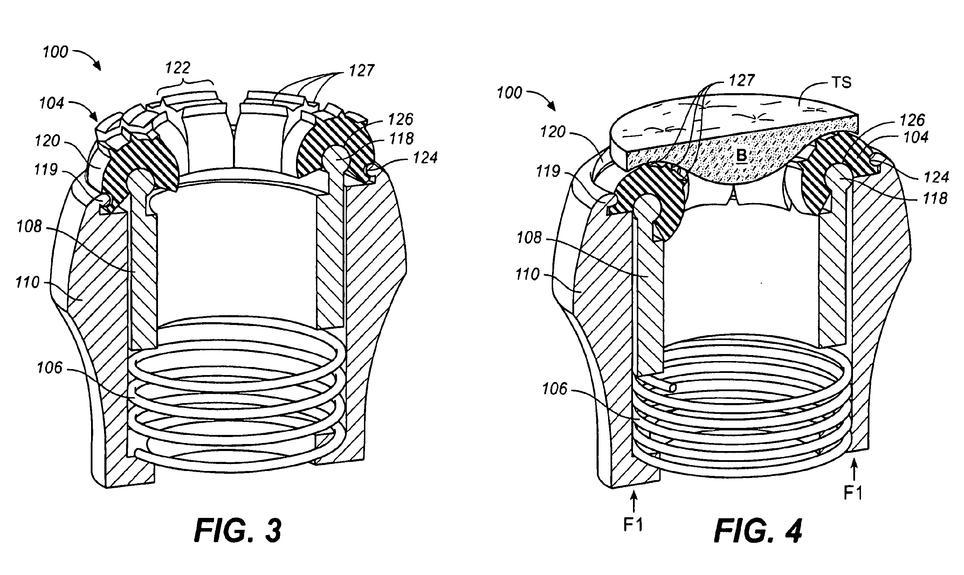

FIG. 1 is a simplified perspective view of a cap 100 for use with a dermal tissue lancing device (not shown) according to an exemplary embodiment of the present invention. FIGs. 2 and 3 are a simplified exploded perspective view and a simplified perspective, partially-cut-away view of cap 100, respectively. FIG. 4 is a simplified, perspective, partially-cut-away view of cap 100 urged against a dermal tissue target site TS such that a target site bulge (B) has been formed.

Referring to FIGs. 1-4, cap 100 includes a retainer 102, a generally ring-shaped segmented cap body 104 and a spring 106. Retainer 102 includes an inner retainer portion 108, an outer retainer portion 110 and an opening 112 along a longitudinal axis A-A (see FIG. 2) of cap 100. Retainer 102 has a proximal end 114 configured for engagement with the dermal tissue lancing device (not shown) and a distal end 116. Furthermore, inner retainer portion 108 includes cap body engagement features 118 and outer retainer portion 110 includes a lip 119.

Proximal end 114 is configured for engagement with the dermal tissue lancing device. For example, proximal end 114 can be removeably attached to an end of a suitably modified conventional lancing device by slideably mounting, snap-fitting or screw-fitting proximal end 114 to the end of the dermal tissue lancing device. One skilled in the art can readily modify suitable conventional dermal tissue lancing devices for engagement with a proximal end of caps according to embodiments of the present invention. Suitable conventional dermal tissue lancing devices are described in, for example, U.S. Patent No.s 5,730,753, 6,045,567 and 6,071,250 .

However, once apprised of the present invention, one skilled in the art will appreciate that caps according to embodiments of the present invention are not limited to use with the dermal tissue lancing devices described in the aforementioned patents. Rather, caps according to embodiments of the present invention can be used with any suitable dermal tissue lancing device including, for example, those that employ lancets, hollow needles, solid needles, micro-needles, ultrasonic devices, thermal techniques, and any other suitable technique for extraction of a bodily fluid sample from a dermal tissue target site. In addition, the dermal tissue lancing device can, if desired, include an integrated analytical system for the determination of an analyte (e.g., glucose) in an expressed bodily fluid sample.

Each of the segments of ring-shaped segmented cap body 104 (i.e., cap body segments 122 noted below), includes a distal compression surface 120, borders opening 112 and is revolvingly engaged with a cap body engagement feature 118 and securely engaged with lip 119. Ring-shaped segmented cap body 104 includes a plurality of cap body segments 122 (namely eight cap body segments 122), an outer recess 124, and an inner recess 126. In addition, ring-shaped segmented cap body 104 includes a plurality of dermal tissue engagement features 127 (also referred to as "ridges" 127) on distal compression surface 120. Although, for the purpose of explanation only, eight cap body segments are depicted in the ring-shaped segmented cap body of FIGs. 1-4, any suitable number of cap body segments can be employed.

Ridges 127 serve to enhance purchase between cap body distal compression surface 120 and a dermal tissue target site. Such enhanced purchase can also be achieved, for example, by forming ring-shaped segmented cap body 104 of a material that is suitably tacky and/or a material that has a suitable high coefficient of friction. An example of such a material is a silica-filled silicone elastomer. Furthermore, enhanced purchase can be achieved via a roughened distal compression surface or a distal compression surface with recesses.

Ring-shaped segmented cap body 104 can be formed of any suitable material including, but not limited to, rigid materials, elastomeric materials, polymeric materials, polyurethane materials, latex materials, silicone materials and combinations thereof. It should be noted that the segmented nature of ring-shaped segmented cap body 104 provides for each cap body segment 122 to revolve about cap body engagement features 118 independently of any other cap body segment and regardless of whether the cap body segments are formed of a rigid or deformable material.

FIGs. 3 and 4 depict the manner in which outer recess 124 of ring-shaped segmented cap body 104 provides for secure engagement with lip 119 of outer retainer portion 110 and inner recess 126 of ring-shaped segmented cap body 104 for secure yet revolving engagement with cap body engagement features 118 of inner retainer portion 108.

As is explained in further detail herein, when a force is exerted on distal compression surface 120 by the urging of cap 100 against a dermal tissue target site, at least a portion of the ring-shaped segmented cap body 104 revolves while ring-shaped segmented cap body 104 remains securely engaged within outer retainer portion 110 by lip 119. This revolution is evident from a comparison of the relative locations of ring-shaped segmented body 104 in FIGs. 3 and 4. The revolution occurs essentially about the circular axis of ring-shaped segmented cap body 104, i.e., about cap body engagement features 118.

Cap body segments 122 essentially rest on cap body engagement features 118 and can revolve thereon. Ring-shaped segmented cap body 104 has a generally C-shaped cross-section (see FIGs. 3 and 4). Once apprised of the present disclosure, one skilled in the art will recognize that although ring-shaped segmented cap body 104 can be generally described as "ring-shaped," such a shape refers to the overall shape of the plurality of cap body segments 122 (each with an inner recess 126, an outer recess 124 and ridges 127). Such a ring-shape can also be generally considered a "toroid" shape or a "doughnut" shape.

Opening 112 can have any suitable cross-sectional shape(s) in a direction perpendicular to longitudinal axis A-A including, but not limited to, circular, square, hexagonal, octagonal and triangular cross-sectional shapes. In addition, the cross-section shape can be such that access to opening 112 by, for example, a test strip is provided. Such test strip access enables beneficial *in-situ* transfer of a blood sample to the test strip as described in U.S. Patent Application 10/143,399 (published as US 2003/0143113 A2 on July 31, 2003 ), International Application No. PCT/US01/07169 (published as WO 01/64105 A1 on September 7, 2001) and International Application No. PCT/GB02/03772 (published as WO 03/015627 A2 on February 27, 2003).

Referring to FIGs. 3 and 4, as cap 100 is urged against a dermal tissue target site by application of force F1, ridges 127 engage the dermal tissue target site. As F1 increases, spring 106 is depressed due to longitudinal relative movement of inner and outer retainers portions 108 and 110 (compare FIGs. 3 and 4). The urging of cap 100 against dermal tissue target site TS results in a torsional force being applied to ring-shaped segmented cap body 104 that results in revolution (i.e., rotation) of ring-shaped segmented cap body 104 (namely cap body segments 122) about cap body engagement features 118. This revolution is inward with respect to opening 112.

During this inward revolution/rotation, ridges 127 and distal compression surface 120 further engage the dermal tissue target site and form a target site bulge B within opening 112 (see FIG. 4). Continued application of force F1 to the dermal tissue target site increases pressure within target site bulge B and, following lancing of target site bulge B, facilitates expressions of bodily fluid (e.g., blood) out of the lanced target site without additional manual manipulation of the lanced target site.

To increase bodily fluid expression, the applied force can be maintained for a predetermined time period (i.e., a post-lance pressure time period) after lancing (e.g., a post-lance pressure time period in the range of about 2 seconds to about 12 seconds). The amount of expressed bodily fluid can also be increased by also applying and maintaining force prior to lancing (i.e., pre-lance pressure) for a predetermined time period, for example, in the range of 1 seconds to 8 seconds and typically in the range between about 3 seconds and 5 seconds.

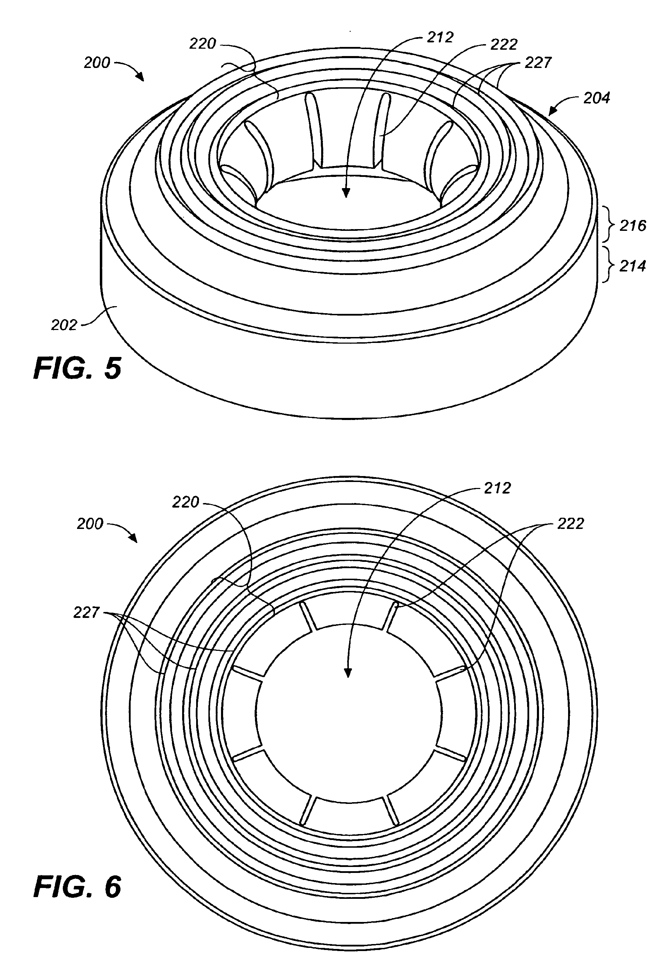

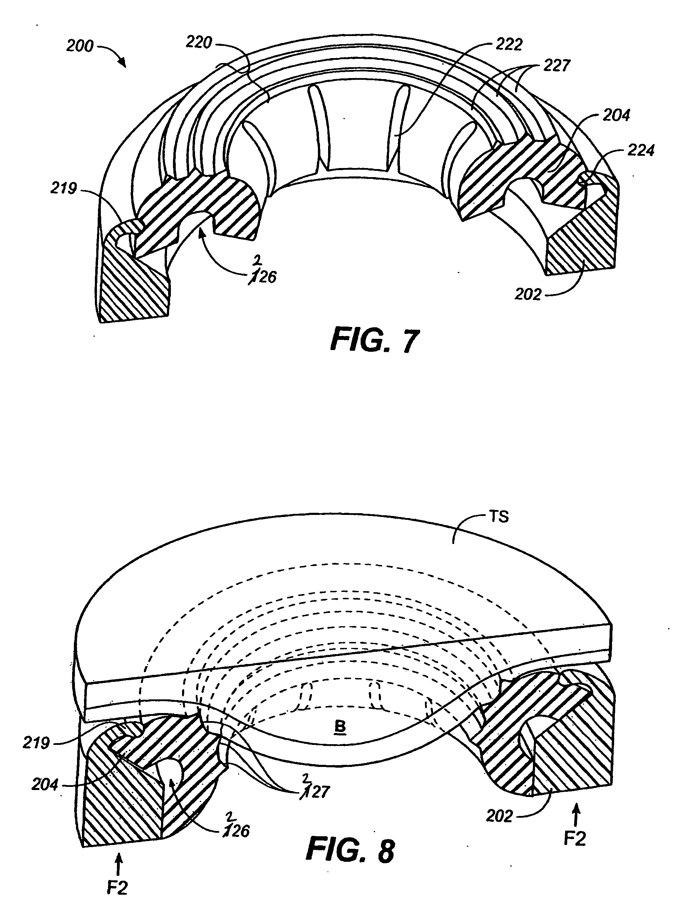

FIG. 5 is a simplified perspective view of a cap 200 for a dermal tissue lancing device cap (not shown) according to another exemplary embodiment of the present invention. FIGs. 6 and 7 are a simplified exploded perspective view and a simplified perspective, partially-cut-away view of cap 200, respectively. FIG. 8 is a simplified, perspective, partially-cut-away view of cap 200 urged against a dermal tissue target site TS such that a target site bulge (B) has been formed.

Referring to FIGs. 5-8, cap 200 includes a retainer 202 and a generally ring-shaped deformable cap body 204. Retainer 202 includes an opening 212 along the longitudinal axis of cap 200. Retainer 202 has a proximal end 214 configured for engagement with the dermal tissue lancing device (not shown) and a distal end 216. Furthermore, retainer portion 202 includes a lip 219.

Ring-shaped deformable cap body 204, including a distal compression surface 220, borders opening 212 and is revolvingly engaged with retainer 202. Ring-shaped deformable cap body 204 includes further a plurality of slits 222, an outer recess 224, and an inner recess 226. In addition, ring-shaped deformable cap body 204 includes a plurality of dermal tissue engagement features 227 (also referred to as "ridges" 227) on distal compression surface 220.

FIGs. 7 and 8 depict the manner in which outer recess 224 of ring-shaped deformable cap body 204 provides for secure engagement with retainer lip 219 while providing for ring-shaped deformable cap body 204 to revolve (i.e., rotate inward) during use (as is evident from a comparison of the position of ring-shaped deformable cap body 204 in FIGs. 7 and 8).

As is explained in further detail herein, when a force is exerted on distal compression surface 220 by the urging of cap 200 against a dermal tissue target site, at least a portion of the ring-shaped deformable cap body 204 revolves while ring-shaped deformable cap body 204 remains securely engaged within retainer 202. The revolution occurs essentially about the circular axis of ring-shaped deformable body 204. Such revolution can be likened to a rotational flexing of the ring-shaped deformable cap body.

Referring to FIGs. 7 and 8, as cap 200 is urged against a dermal tissue target site by application of force F2, ridges 227 engage the dermal tissue target site. The urging of cap 200 against dermal tissue target site TS (and a radially outward retaining effect of retainer 202) results in a torsional force being applied to ring-shaped deformable cap body 204 that causes revolution (i.e., rotation) of ring-shaped deformable cap body 204 inward with respect to opening 212. Slits 222 and inner recess 226 facilitate such revolution while retainer 202 serves to limit radially outward movement of ring-shaped deformable cap body 204.

During this inward revolution/rotation, ridges 227 and distal compression surface 220 further engage the dermal tissue target site and form a target site bulge B within opening 212 (see FIG. 8). Continued application of force F2 to the dermal tissue target site increases pressure within target site bulge B and, following lancing of target site bulge B, facilitates expressions of bodily fluid (e.g., blood) out of the lanced target site without additional manual manipulation of the lanced target site.

During use, there is a potential for dermal tissue lancing device caps to come into contact with blood or other bodily fluid. Such contact could conceivably lead to contamination of the cap with micro-organisms (e.g., bacteria or fungi) or viruses of undesirable activity. However, caps according to embodiments of the present invention can be optionally formed, at least partially, of a suitable anti-microbial material, antifungal material and/or anti-viral material that serves to alleviate the undesirable activity of such micro-organisms or viruses. Such a suitable material can be, for example, an anti-microbial plastic, anti-microbial resin and/or anti-microbial silicone. Suitable anti-microbial materials can include, for example, anti-microbial compounds with a trichloro-phenol group, such as 2, 4, 4 -trichloro-2-hydroxy diphenol ether. The anti-microbial compound can be, for example, a coating of the cap or incorporated directly in the cap.

Based on the description of caps 100 and 200 above, one skilled in the art will recognize that caps according to embodiments of the present invention generally include a retainer and a ring-shaped cap body (such as, a ring-shaped deformable cap body or a ring-shaped segmented cap body). Moreover, the retainer has a proximal end configured for engagement with the dermal tissue lancing device, a distal end with a cap body engagement feature (such as a lip) and an opening. In addition, the ring-shaped cap body has a distal compression surface, borders the opening and is securely and revolvingly engaged with the cap body engagement feature. Also, when a force is exerted on the distal compression surface during use of the cap, the ring-shaped cap body revolves (e.g., inward with respect to the opening) while remaining securely engaged with the retainer.

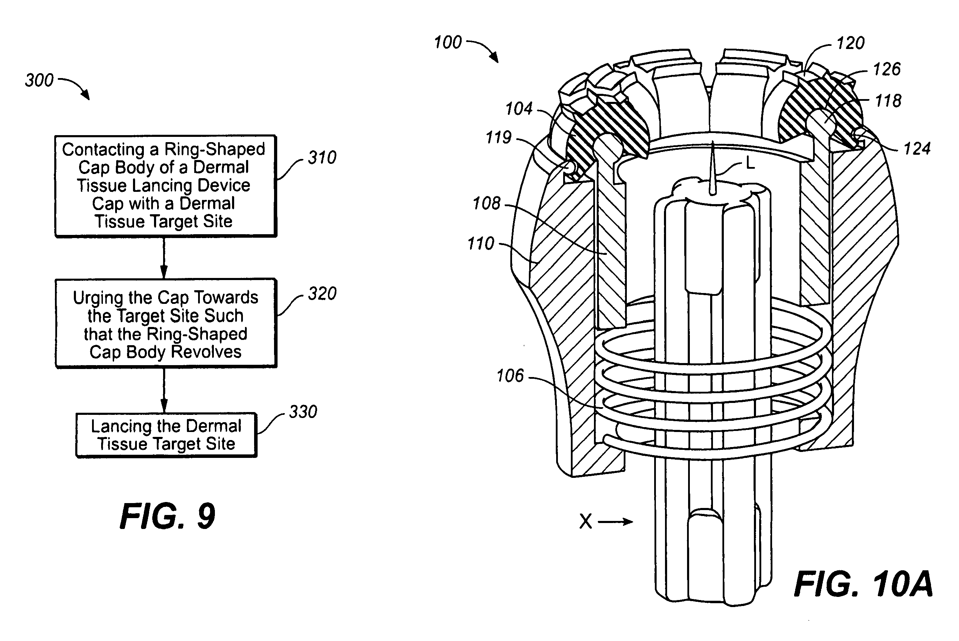

FIG. 9 is a flow chart illustrating a sequence of steps in a process 300 for lancing a dermal tissue target site TS using a cap with a revolving cap body. FIGs. 10A through 10C are simplified cross-sectional views depicting various stages of the process of FIG. 9. For illustrative purposes, cap 100 of FIG. 1 is depicted in FIGs. 10A-10C as being employed in process 300. However, one skilled in the art will recognize that any cap for a dermal tissue lancing device according to the present invention can be employed in methods for lancing a dermal tissue target site according to the present invention. In this regard, it should be noted that any functional behavior or use of caps for dermal tissue lancing devices according to embodiments of the present invention as described herein can be included in methods for lancing a dermal tissue target site according to the present invention. Moreover, one skilled in the art will recognize that FIGs. 10A through 10C depict only a portion X of a dermal tissue lancing device with portion X including a lancet L.

Process 300 includes for contacting a distal compression surface 120 of a ring-shaped segmented cap body 104 of a dermal tissue lancing device cap 100 with the dermal tissue target site TS (see step 310 of FIG. 9, with FIG. 10A depicting dermal tissue lancing device cap 100 prior to use). Although, for the purpose of explanation only, process 300 is described in conjunction with a ring-shaped segmented cap body, processes according to embodiments of the present invention can generally employ any suitable ring-shaped cap body including, for example, ring-shaped deformable cap body 204.

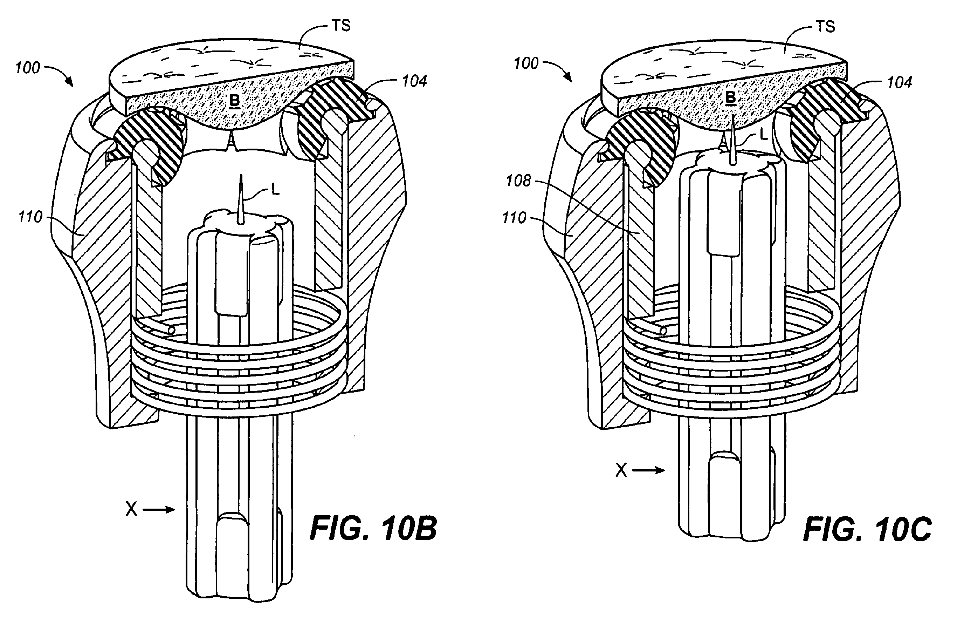

The dermal tissue lancing device cap 100 is then urged towards the dermal tissue target site TS, such that a force is exerted on the distal compression surface 120 that results in the ring-shaped segmented cap body 104 revolving while remaining securely engaged within a retainer (i.e., retainer inner and outer portions 108 and 110, respectively) of the dermal tissue lancing device cap 100. See step 320 of FIG. 9 and FIG. 10B.

Subsequently, a target site bulge B of the dermal tissue target site TS is lanced with lancet L of the dermal tissue lancing device, as set forth in step 330 of FIG. 9 and as illustrated in FIG. 10C.

**Example** - Comparative Cap Success Rate and Subjective Discomfort

A comparative study between a cap for a dermal tissue lancing device according to an embodiment of the present invention (i.e., cap 200 of FIGs. 5, 6, 7 and 8) and a conventional rigid cap was conducted using a 0.320 mm (28-gauge) lancet available from Becton Dickinson of Franklin Lakes, NJ.

The method of testing comprised pressing the cap body (fitted onto the distal end of a conventional lancing device) against a dermal tissue target site of a subject's finger for 3 seconds, lancing the dermal tissue target site with a 0.320 mm (28-gauge) needle, continuing to hold the cap against the dermal tissue target site for 10 seconds, removing the cap from the dermal tissue target site and collecting blood from the lanced dermal tissue target site with a calibrated glass capillary pipette.

During the lancing step, the subjects rated the amount of discomfort experienced using a subjective scale ranging from 0 to 10. In this subjective scale, a rating of 0 indicated that the subject did not feel any pain during lancing and a rating of 10 indicating that lancing was very painful to the subject. The average subjective score for cap 500 was 2.5 versus 4.3 for the rigid cap. This score indicates that the level of discomfort associated with use of cap 200 is relatively low.

Success was defined as obtaining at least 0.7 microliters of blood (i.e., typically the minimum volume required to give an accurate assessment of an analyte, such as glucose, in blood with hand-held devices). Percent success rate is given for all cap designs tested in Table I below (where n is the number of subjects tested).

**TABLE 1**

| **Pressure Caps** | **Volume, uL Mean ± SD** | **Subjective Discomfort Mean ± SD** | **% Success Rate ≥ 0.7 uL** |
|---|---|---|---|
| Cap 200 (n=36) | 2.1 ± 1.4 | 2.5 ± 1.7 | 89 |
| Rigid Cap (n=36) | 0.0 ± 0.0 | 4.2 ± 2.0 | 0 |

The data in Table I indicate a significant percent success rate with cap 200 when compared to the rigid cap. Since the dermal tissue target sites were not physically manipulated (other than by the caps themselves as described above) to enhance blood expression, the success rate indicates that caps according to embodiments of the present invention do not require physical manipulation for blood expression.

It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A cap (100) for a dermal tissue lancing device, the cap comprising:
a retainer (102) with:
a proximal end (114) configured for engagement with the dermal tissue lancing device; and
a distal end (116) with at least one cap body engagement feature (118, 202);
an opening (112) through the retainer along a longitudinal axis of the cap;
a ring-shaped segmented cap body (104) with a distal compression surface (120),
wherein the ring-shaped segmented cap body borders the opening and is securely and revolvingly engaged with a cap body engagement feature, and
wherein force exerted on the distal compression surface during use of the cap results in at least a portion of the ring-shaped segmented cap body revolving while remaining securely engaged with the retainer,
wherein the ring-shaped segmented cap body has a generally toroidal shape.

2. The cap of claim 1, wherein the ring-shaped segmented cap body has a generally C-shaped cross-section.

3. The cap of claim 1 or 2, wherein the ring-shaped segmented cap body defines a circular axis about which at least one said portion is revolvable.

4. The cap of any preceding claim, wherein the ring-shaped segmented cap body includes a plurality of cap body segments and each of the cap body segments is revolvingly engaged with a cap body engagement feature.

5. The cap of claim 4, wherein the plurality of cap body segments are formed of a rigid material.

6. The cap of any preceding claim, wherein the retainer includes an inner retainer portion and outer retainer portion and the at least one cap body engagement feature includes a lip of the outer retainer portion.

7. The cap of any preceding claim, wherein the ring-shaped segmented cap body revolves inward towards the opening.

8. The cap of any preceding claim, wherein the ring-shaped segmented cap body includes a plurality of cap body segments and the cap body segments revolve inward toward the opening,

9. The cap of any preceding claim, wherein the segmented cap body is at least partially formed of an anti-microbial material.

10. The cap of claim 9, wherein the anti-microbial material includes a trichloro-phenol compound.

11. The cap of claim 10, wherein the trichloro-phenol compound is 2,4,4-Trichloro-2-hydroxy-diphenol ether.

12. The cap of any preceding claim, wherein the segmented cap body distal compression surface has a plurality of ridges disposed thereon.

13. The cap of any preceding claim, wherein the retainer is configured to limit radially outward movement of the ring-shaped deformable segmented cap body during use of the cap.

14. The cap of any preceding claim, wherein the ring-shaped segmented cap body is a ring-shaped deformable cap body with a plurality of slits.

## Patentansprüche

1. Kappe (100) für eine Hautgewebe-Lanzettenvorrichtung, wobei die Kappe Folgendes umfasst:
einen Halter (102) mit:
einem proximalen Ende (114), das zum Eingriff mit der Hautgewebe-Lanzettenvorrichtung konfiguriert ist; und
einem distalen Ende (116) mit mindestens einer Kappenkörper-Eingriffseinrichtung (118, 202);
einer Öffnung (112) durch den Halter entlang einer Längsachse der Kappe;
einem ringförmigen segmentierten Kappenkörper (104) mit einer distalen Kompressionsoberfläche (120),
wobei der ringförmige segmentierte Kappenkörper die Öffnung umrandet und mit einer Kappenkörper-Eingriffseinrichtung sicher und drehbar in Eingriff steht, und
wobei die Kraft, die während des Gebrauchs der Kappe auf die distale Kompressionsoberfläche ausgeübt wird, dazu führt, dass sich mindestens ein Abschnitt des ringförmigen segmentierten Kappenkörpers dreht, während er mit dem Halter sicher in Eingriff bleibt,
wobei der ringförmige segmentierte Kappenkörper eine im Allgemeinen toroidale Form aufweist.

2. Kappe nach Anspruch 1, wobei der ringförmige segmentierte Kappenkörper einen im Allgemeinen C-förmigen Querschnitt aufweist.

3. Kappe nach Anspruch 1 oder 2, wobei der ringförmige segmentierte Kappenkörper eine kreisförmige Achse definiert, um die der mindestens eine Abschnitt drehbar ist.

4. Kappe nach einem der vorherigen Ansprüche, wobei der ringförmige segmentierte Kappenkörper mehrere Kappenkörpersegmente aufweist und jedes der Kappenkörpersegmente mit einer Kappenkörper-Eingriffseinrichtung drehbar in Eingriff steht.

5. Kappe nach Anspruch 4, wobei die mehreren Kappenkörpersegmente aus einem steifen Material gebildet sind.

6. Kappe nach einem der vorherigen Ansprüche, wobei der Halter einen inneren Halterabschnitt und einen äußeren Halterabschnitt aufweist und die mindestens eine Kappenkörper-Eingriffseinrichtung einen Rand des äußeren Halterabschnitts aufweist.

7. Kappe nach einem der vorherigen Ansprüche, wobei sich der ringförmige segmentierte Kappenkörper nach innen zur Öffnung dreht.

8. Kappe nach einem der vorherigen Ansprüche, wobei der ringförmige segmentierte Kappenkörper mehrere Kappenkörpersegmente aufweist und sich die
Kappenkörpersegmente nach innen zur Öffnung drehen.

9. Kappe nach einem der vorherigen Ansprüche, wobei der segmentierte Kappenkörper mindestens teilweise aus einem antimikrobiellen Material gebildet ist.

10. Kappe nach Anspruch 9, wobei das antimikrobielle Material eine Trichlorphenolverbindung enthält.

11. Kappe nach Anspruch 10, wobei die Trichlorphenolverbindung 2,4,4-Trichloro-2-hydroxy-diphenolether ist.

12. Kappe nach einem der vorherigen Ansprüche, wobei auf der distalen Kompressionsoberfläche des segmentierten Kappenkörpers mehrere Kämme angeordnet sind.

13. Kappe nach einem der vorhergehenden Ansprüche, wobei der Halter derart konfiguriert ist, dass er eine Bewegung des ringförmigen segmentierten Kappenkörpers während des Gebrauchs der Kappe radial nach außen begrenzt.

14. Kappe nach einem der vorherigen Ansprüche, wobei der ringförmige segmentierte Kappenkörper ein ringförmiger verformbarer Kappenkörper mit mehreren Schlitzen ist.

## Revendications

1. Capuchon (100) pour un dispositif auto-piqueur de tissus dermiques, comprenant:
un dispositif de retenue (102) présentant:
une extrémité proximale (114) qui est configurée de manière à s'engager avec le dispositif auto-piqueur de tissus dermiques; et
une extrémité distale (116) présentant au moins une caractéristique d'engagement de corps de capuchon (118, 202);
une ouverture (112) à travers le dispositif de retenue le long d'un axe longitudinal du capuchon;
un corps de capuchon segmenté de forme annulaire (104) qui présente une surface de compression distale (120),
dans lequel le corps de capuchon segmenté de forme annulaire borde l'ouverture et est engagé de façon sûre et rotative avec une caractéristique d'engagement de corps de capuchon, et
dans lequel la force qui est exercée sur la surface de compression distale pendant l'utilisation du capuchon entraîne qu'au moins une partie du corps de capuchon segmenté de forme annulaire tourne tout en restant engagée de façon sûre avec le dispositif de retenue,
dans lequel le corps de capuchon segmenté de forme annulaire présente une forme essentiellement toroïdale.

2. Capuchon selon la revendication 1, dans lequel le corps de capuchon segmenté de forme annulaire présente une section transversale essentiellement en forme de C.

3. Capuchon selon la revendication 1 ou 2, dans lequel le corps de capuchon segmenté de forme annulaire définit un axe circulaire autour duquel ladite au moins une partie peut tourner.

4. Capuchon selon l'une quelconque des revendications précédentes, dans lequel le corps de capuchon segmenté de forme annulaire comprend une pluralité de segments de corps de capuchon, et chacun des segments de corps de capuchon est engagé de façon rotative avec une caractéristique d'engagement de corps de capuchon.

5. Capuchon selon la revendication 4, dans lequel la pluralité de segments de corps de capuchon sont constitués d'un matériau rigide.

6. Capuchon selon l'une quelconque des revendications précédentes, dans lequel le dispositif de retenue comprend une partie de retenue intérieure et une partie de retenue extérieure, et ladite au moins une caractéristique d'engagement de corps de capuchon comprend une lèvre de la partie de retenue extérieure.

7. Capuchon selon l'une quelconque des revendications précédentes, dans lequel le corps de capuchon segmenté de forme annulaire tourne vers l'intérieur en direction de l'ouverture.

8. Capuchon selon l'une quelconque des revendications précédentes, dans lequel le corps de capuchon segmenté de forme annulaire comprend une pluralité de segments de corps de capuchon, et les segments de corps de capuchon tournent vers l'intérieur en direction de l'ouverture.

9. Capuchon selon l'une quelconque des revendications précédentes, dans lequel le corps de capuchon segmenté est au moins partiellement constitué d'un matériau antimicrobien.

10. Capuchon selon la revendication 9, dans lequel le matériau antimicrobien comprend un composé de trichloro-phénol.

11. Capuchon selon la revendication 10, dans lequel le composé de trichloro-phénol est un 2,4,4-trichloro-2-hydroxy-diphénol éther.

12. Capuchon selon l'une quelconque des revendications précédentes, dans lequel la surface de compression distale du corps de capuchon segmenté comporte une pluralité de nervures qui sont disposées sur celle-ci.

13. Capuchon selon l'une quelconque des revendications précédentes, dans lequel le dispositif de retenue est configuré de manière à limiter le déplacement radialement vers l'extérieur du corps de capuchon segmenté déformable de forme annulaire pendant l'utilisation du capuchon.

14. Capuchon selon l'une quelconque des revendications précédentes, dans lequel le corps de capuchon segmenté de forme annulaire est un corps de capuchon déformable de forme annulaire qui comporte une pluralité de fentes.
